# EUROPEAN PATENT SPECIFICATION

(11) **EP 1 250 169 B1**
(45) Date of publication and mention of the grant of the patent: **09.03.2005**
(21) Application number: 00957151.4
(22) Date of filing: 31.08.2000
(51) Int. Cl.: A61M 16/00

(54) **AN ACCESSORY DEVICE FOR A RESUSCITATION UNIT**
ZUBEHÖR FÜR EINE WIEDERBELEBUNGSEINHEIT
DISPOSITIF ASSOCIE A UNE UNITE DE REANIMATION

(30) Priority: 01.09.1999 NO 994230
(43) Date of publication of application: 23.10.2002
(73) Proprietor: Lovstad, Frank, 3150 Tolvsrod (NO)
(72) Inventor: Lovstad, Frank, 3150 Tolvsrod (NO)
(74) Representative: Onn, Thorsten
(86) International application number: PCT/NO2000/000284
(87) International publication number: WO 2001/015761

(56) References cited:
- US-A- 4 196 725
- US-A- 4 297 999
- US-A- 5 497 767
- US-A- 5 875 777
- US-A- 5 931 162

## Description

The present invention relates to an accessory device for a resuscitation unit. More precisely, the invention relates to a device for use in connection with conventional resuscitation equipment to enhance safety during the ventilation treatment of premature and neonatal infants.

Broncopulmonary dysplasia, BDP, is a highly common and dreaded complication in premature infants, i.e., infants bom before term, whose lungs are not yet fully developed (surfactant deficiency, hyaline membrane syndrome, respiratory distress and so forth).

When there is surfactant deficiency, the lungs have a tendency to develop progressive atelectasis, which in turn results in the need for respirator support, often with increasing pressure support.

Often, a lung deficient in surfactants will also require ventilation by means of resuscitation equipment before the infant is placed in the respirator.

For this first phase whilst the infant is ventilated by bag, there is today no reliable control of the pressure used during the ventilation or of the gas volume with which the infant's lungs are filled.

Although the conventional resuscitation bags that are currently available have a so-called safety valve that should be released at a pressure of about 30 cm H₂O, it is known today that this does not always happen.

Studies carried out at Temple University in Philadelphia and also at the neonatal intensive care unit at Ullevål Hospital in Oslo have shown that the bags used today and which are generally available on the market, do not release the safety valve until the pressure is over 30 cm H₂O, and at ventilation of frequencies ≥ 80/min, release often only takes place when the pressure is ≥ 50 cm H₂O.

The reason that premature infants develop BPD is so-called baro-trauma and/or volu-trauma, i.e., that both the pressure and the volume used are greater than the lungs of the smallest premature infants can withstand. Today, there is much to suggest that the infant suffers these traumas as early as in the delivery ward, or perhaps in the operating theatre if delivery by Caesarean section is required, when the infant is in need of resuscitation.

Over the years a great number of publications have appeared, including some from the aforementioned medical centres, that stress the importance of reducing bare-trauma in the neonatal period in order to prevent chronic lung disease. It is believed that this problem affects about 37% of small, premature infants that have recovered after severe respiratory distress syndrome at birth.

Today, therefore, efforts are being made to minimise the pressure exposure to which infants are subjected in all phases of neonatal treatment.

Consequently, there is an explicit need among those wishing to make the treatment, including resuscitation, of premature and neonatal infants, as safe as possible, and therefore a device is required that can be used together with today's conventional resuscitation bags to safeguard against baro-trauma and volu-trauma by reacting both quickly and reliably when predetermined maximum values are exceeded.

The object of the present invention is to remedy the deficiencies of the prior art, and the invention is in principle based on the following ideas.

Today, in the resuscitation of neonatal or premature infants, a manual ventilation bag is used. In premature births from 24 weeks of gestation to 36 weeks inclusive, the infant's airways, as mentioned above, are not fully developed, and these infants, who cannot yet breathe unaided, will be hand-ventilated with a ventilation bag in the first important minutes after birth.

Because the airways, as already mentioned, are not fully developed, they are also extremely sensitive as they lack the essential elasticity, primarily because the natural surface-active agents, surfactants, which contribute greatly to the elasticity are lacking.

It is a fact that in today's means for immediate hand ventilation, there is no full control of the pressure, volumes and frequencies that are used. When an infant in such a situation does not breathe, the staff often lose their sense of time to a certain degree, even highly experienced staff. This may unconsciously result in an excessively high ventilation frequency.

Depending somewhat on local routines, a mechanical manometer may be connected to measure the pressure with which the newborn infant is ventilated.

The pressure is measured at the connection between the bag and the face mask. However, the problem with this technology is that the manometers used do not react anywhere near quickly enough, and therefore the medical personnel have no true picture of the pressure that in fact prevails.

If a faster reacting digital pressure recorder is connected, a substantially higher pressure will be seen when the frequency rises.

However, there is also no limit on the pressure if the staff are a little careless or inexperienced and may thus inadvertently ventilate using an excessively high pressure.

Lastly, there is no control as regards the volume delivered. Here it should be remembered that the volumes involved are as little as 2 to 3 ml.

There are pressure relief valves on the market, available from such suppliers as Siemens, Dragerwerk and Sechrist, that can be fitted on the ventilation bag. These are mechanical spring valves which have a very poor frequency response, i.e., in the first place, they are highly inaccurate as regards the pressure and, in the second place, the pressure released increases at frequencies above 40/min.

On account of these factors as they are summarised above, there have been cases of ventilation frequencies as high as 120/min.

There are also breathing assist devices which include both pressure and flow meters, such as the one disclosed in US 5,875,777. The measurement signals from the sensors are in this device used for imposing a mechanical breathing pattern, corresponding to the manually generated breathing pattern, on the patient. No relief valves are used for ensuring that the pressure or the volume delivered does not become too high.

All these factors separately and, of course, even more so in combination, will result in hyperventilation (an overstretching of the infant's airways). If there is such an overstretching of the rather inelastic parts of the airways, these extremely small airways (alveoli) will not return to their original form but will instead pass into a plastic state, which at worst can result in death, and at best can result in the child remaining in a respirator for days, weeks or even months.

As mentioned above, it is therefore an express wish among those who work with premature and neonatal infants to have at their disposal a simple product that in a simple manner limits the pressure and volume and which at the same time gives clear indications as regards the ventilation frequency that is to be used.

Thus, the object of the present invention is to develop a device this kind that also should be capable of being connected promptly and readily to existing resuscitation equipment.

The idea behind the present invention is to develop a simple monitor that registers and regulates the pressure and volume of air that is given to premature infants by hand ventilation. This monitor must have an external sensor that is connected to the manual ventilation bag (or the bellows) used, between the bag and the face mask.

When the volume or the pressure reaches a given value the device should "blow off" the excess.

One way of doing this is to work with an electromagnetic pressure control valve.

Advantageously, the valve is self-regulating, i.e., just one signal to the valve will be sufficient to ensure that it carries out the required control.

Accordingly, the present invention relates to an accessory device as defined in claim 1.

The invention will be explained in more detail with the use of the attached figures, wherein:
- Figure 1 is a block diagram of the electronic limitation of hand ventilation;
- Figure 2 is an outline of an embodiment of the pathway for the electronic signals;
- Figure 3 is a schematic diagram of an electromagnetic pressure/flow control valve; and
- Figure 4 shows what a control box may look like.

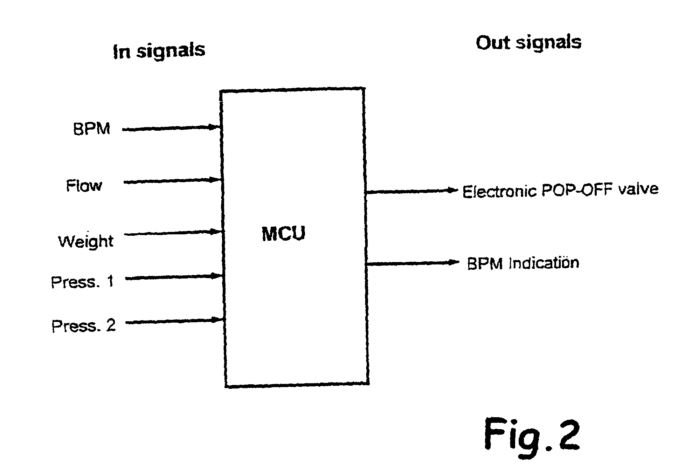

In the figures the letters MCU stand for "Micro Control Unit". In Figure 2 the letters BPM stand for the breathing frequency "Breath per Minute".

The inventive idea is based on the principle of installing a control box that is to measure flow and pressure and on the basis thereof compute inhaled volume and pressure. When the correct volumes or pressures have been reached, the operating pressure is to be reduced so that inspiration is terminated.

As mentioned above, this is intended as a complement to the known manual ventilation equipment for premature and neonatal patients, as premature infants have a special need for accurate control of the ventilation in order to prevent the damage outlined above from occurring.

In principle, it is possible to use any rapid-action, self-regulating electronically operated valve, but in a preferred embodiment electromagnetism is used as an actuating force on a magnetically sensitive object to enable the object to close a flow path with desired force and thus be able to control the flow therethrough. As mentioned, it is desirable that the valve should be self-regulating, i.e., that just one signal should be sufficient to enable it to carry out its control functions.

It is also possible to use a solenoid valve.

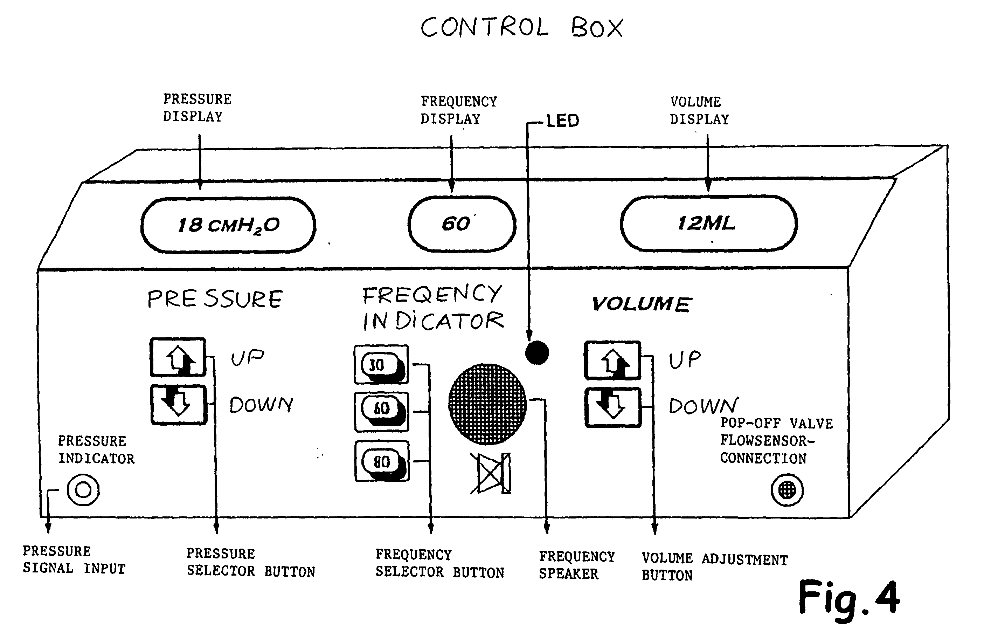

The control box, as indicated in Figure 4, contains a micro-processor that processes the signals it receives from a flow sensor and a pressure sensor. The user himself must define the desired pressure and volume limits within a restricted range, as the conditions must be established instantly, according to all the circumstances of the birth. The received signals then control the further processing of the signals by the micro-processor.

As mentioned above, frequency plays a very important role, and within the given circumstances surrounding the birth it should be possible to pre-set, for example, at most three frequencies which then must be observed during the subsequent hand ventilation.

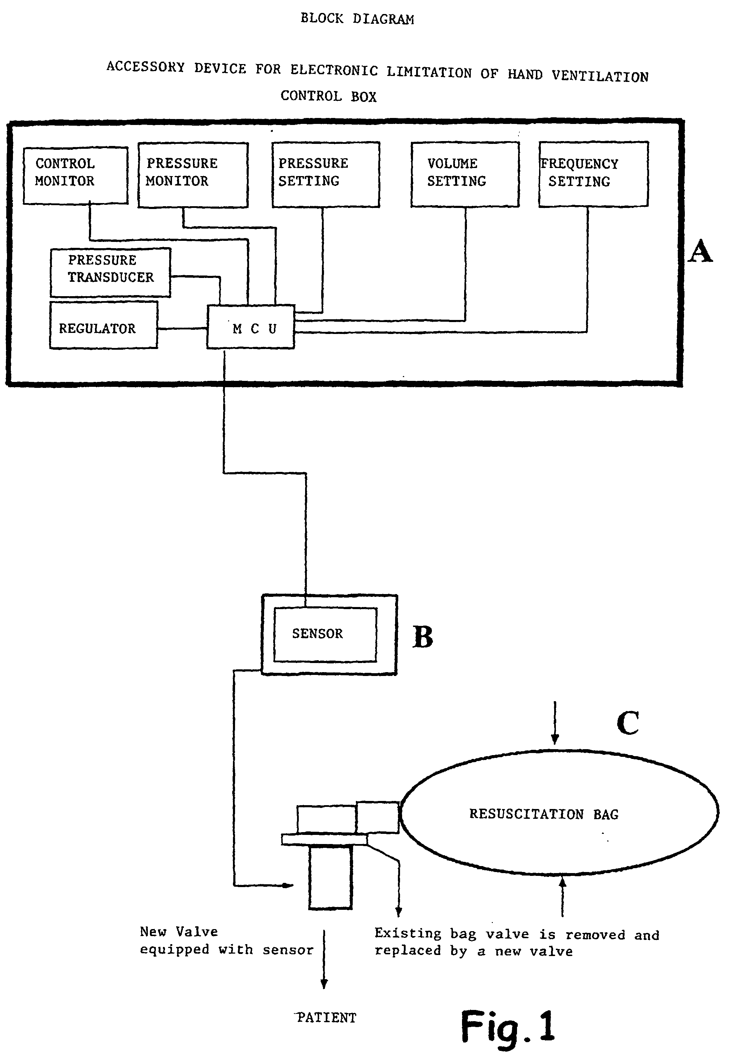

Fig. 1 shows in general the units required to be able to replace an existing bag valve with a valve belonging to the device according to the invention.

Volume and pressure are monitored and maintained against set pressure and volume at the same time as the selected frequency is given.

Figure 2 shows in slightly greater detail a possible connection to the computer unit where the signals are processed. Outgoing signals are also indicated in the drawing.

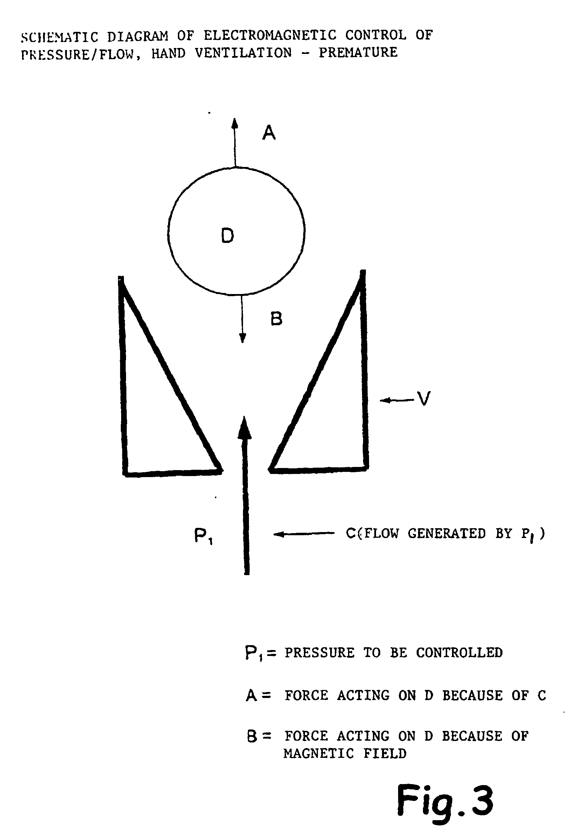

Figure 3 shows a possible embodiment of a valve in the device according to the invention. Flow C under a pressure P1 acts against a closing body D with force A whilst the element D is held in the valve V by means of an applied magnetic field.

Lastly, Figure 4 shows, as mentioned above, a control box which in addition to pressure also contains a setting means for desired ventilation frequency and volume based on the given body weight and in which also flow sensor signals enter.

The box is also equipped with a form of frequency indicator, for example, by using a loudspeaker and/or light emitting diode as shown in the figure.

Broadly speaking, the device consists of two parts, a flow sensor and a control box with equipped with a pressure sensor and control buttons for setting the desired tidal volume, frequency and pressure.

The frequency is to be indicated by a "metronome" or pacer that provides the user with sounds (ticks) and light signals (diode). It is important that there are few frequency choices, for example, 30-60-80.

Volume can be set either by choosing, with the use of a control button, the body weight of the patient, and in so doing be given a pre-programmed volume/kg (for example, 5 ml/kg), or by choosing a number ml tidal volume directly as a value. The last alternative is perhaps the most useful because the first alternative substantially limits the area of application of the equipment.

Maximum inspiratory pressure is selected by means of a separate control button. The selected maximum pressure should then be guiding for the control of the regulator.

Pressure and volume should be equally important controlling parameters (that reached first).

By means of the inventive accessory device, it will be possible to greatly enhance safety during hand ventilation of neonatal and premature infants.

The device is easy to make, and it can be varied according to the circumstances surrounding the birth. It will provide a rapid reaction and contain few details that can be harmed or destroyed.

An essential feature is that the device can be connected to all hand ventilation bags in existence today as devices of this kind have standard connection units.

A major advantage is that the equipment is easy to use and that it is inexpensive to procure and easy to maintain.

## Claims

1. Accessory device, which is connectable to conventional manual resuscitation equipment for neonatal or premature infants, for preventing volutrauma and/or barotrauma in connection with resuscitation in the first phase of life, said device comprising:
a flow sensor for measuring the flow of air to and from the lungs of the infant;
a pressure sensor for measuring the pressure used during resuscitation; and
computing means for computing the tidal volume and the pressure in the airways based on said measured flow and pressure;
**characterized in that** the device further comprises:
a frequency-adjustable unit for indicating rhythm;
means for monitoring and maintaining said computed tidal volume and said computed pressure against preset maximum values; and
a self regulating maximum pressure/volume valve to be arranged between conventional resuscitation equipment and its associated face mask or endotracheal tube (ET tube) for blowing off excess air if either one of the preset maximum values is reached.

2. Device according to claim 1, further comprising:
means for indicating the rhythm through sound and light signals;
a control unit for input of maximum values for pressure or volume;
a display for electronically showing pressure and tidal volume; and
an electronically operated valve for rapid response for blowing off excess volume and pressure of air using the self regulating maximum pressure/volume valve.

## Patentansprüche

1. Zusatzvorrichtung zum Anschluss an herkömmliche manuelle Reanimationsgeräte für Neugeborene oder Frühgeburten zur Vermeidung von Volutraumen und/oder Barotraumen bei der Reanimation in der ersten Lebensphase, enthaltend:
einen Durchflusssensor zur Messung des in die Lunge des Kindes einströmenden und aus derselben ausströmenden Luftstroms;
einen Drucksensor zur Messung des bei der Reanimation verwendeten Drucks; und
Rechenmittel zur Berechnung des Atemvolumens und des Drucks in den Luftwegen auf der Grundlage des gemessenen Durchflusses und Drucks; **dadurch gekennzeichnet, dass** die Vorrichtung weiterhin umfasst:
eine in der Frequenz einstellbare Einheit zur Angabe des Rhythmus;
Mittel zur Überwachung und Einhaltung des berechneten Atemvolumens und des berechneten Drucks gegenüber vorgegebenen Maximalwerten; und
ein selbstregulierendes Maximaldruck/Volumenventil, welches zwischen dem herkömmlichen Reanimationsgerät und der zugehörigen Gesichtsmaske bzw. dem Endotrachealschlauch (ET-Schlauch) eingefügt wird, um überschüssige Luft abzulassen, wenn einer der vorgegebenen Maximalwerte erreicht wird.

2. Vorrichtung nach Anspruch 1, weiterhin enthaltend:
Mittel zur Angabe des Rhythmus durch Ton- und Lichtsignale;
eine Steuereinheit zur Eingabe von Maximalwerten für Druck oder Volumen;
eine Anzeige zur elektronischen Darstellung des Drucks und des Atemvolumens; und
ein elektronisch betriebenes, schnell ansprechendes Ventil zum Ablassen überschüssigen Luftvolumens und von Überdruck über das selbstregulierende Maximaldruck/Volumenventil.

## Revendications

1. Dispositif auxiliaire pour connexion à un appareil conventionnel de réanimation manuel pour nouveaunés ou prématurés, pour éviter des volutraumatismes et/ou barotraumatismes en rapport avec la réanimation dans la première phase de la vie, ledit dispositif comprenant:
un détecteur de débit pour mesurer le débit d'air vers les et en provenance des poumons de l'enfant;
un détecteur de pression pour mesurer la pression utilisée pendant la réanimation; et
des moyens de calcul pour calculer le volume courant et la pression dans les voies respiratoires sur la base dudit débit et de ladite pression mesurés;
**caractérisé en ce que** le dispositif comprend en outre:
une unité réglable en fréquence pour indiquer le rythme;
des moyens pour surveiller et maintenir ledit volume courant calculé et ladite pression calculée dans des valeurs maxima préréglées; et
une soupape autorégulatrice de pression/volume maximum, destinée à être placée entre l'appareil de réanimation conventionnel et le masque facial ou tube endotrachéal (tube ET) y associé afin d'évacuer le surplus d'air lorsque l'une des valeurs maxima préréglées est atteinte.

2. Dispositif selon la revendication 1, comprenant en outre:
des moyens permettant d'indiquer le rythme par des signaux sonores et lumineux;
une unité de commande pour l'entrée de valeurs maxima relatives à la pression ou au volume;
un affichage pour la représentation électronique de la pression et du volume courant; et
une soupape à réponse rapide actionnée par voie électronique pour évacuer le surplus en volume et pression d'air par l'intermédiaire de la soupape autorégulatrice de pression/volume maximum.
